# EUROPEAN PATENT SPECIFICATION

(11) **EP 3 585 291 B1**
(45) Date of publication and mention of the grant of the patent: **24.07.2024**
(21) Application number: 17711829.6
(22) Date of filing: 27.02.2017
(51) Int. Cl.: A61B 18/12, A61B 90/00, A61K 31/00, A61P 25/04

(54) **SYSTEM FOR IMPROVING LOCATION ACCURACY OF A RADIOFREQUENCY ABLATION PROCEDURE VIA FIDUCIAL MARKING**
SYSTEM ZUR VERBESSERUNG DER POSITIONSGENAUIGKEIT EINES HOCHFREQUENZABLATIONSVERFAHRENS DURCH BEZUGSMARKIERUNG
SYSTÈME POUR AMÉLIORER LA PRÉCISION DE LOCALISATION D'UNE INTERVENTION D'ABLATION PAR RADIOFRÉQUENCE PAR L'INTERMÉDIAIRE D'UN MARQUAGE DE REPÈRE

(43) Date of publication of application: 01.01.2020
(73) Proprietor: Avent, Inc., Alpharetta, GA 30004 (US)
(72) Inventor: OLLERENSHAW, Jeremy D., Alpharetta, Georgia 30004 (US); BOURGEOIS, Elliot B., Alpharetta, Georgia 30004 (US)
(74) Representative: Dehns
(86) International application number: PCT/US2017/019603
(87) International publication number: WO 2018/156160

(56) References cited:
- EP-A2- 1 600 113
- WO-A2-2013/154776
- US-A1- 2005 283 148
- US-A1- 2009 024 124
- US-A1- 2014 135 661
- US-A1- 2016 242 661

## Description

### Field of the Invention

The present invention relates generally to systems for treating pain via a nerve block or radiofrequency ablation.

### Background

In the treatment of pain, nerve blocking via a pharmacological agent and radiofrequency (RF) ablation are often conducted as a series of procedures scheduled over a time period of several days or even months. For example, subjecting target nerve to a nerve block via a pharmacological agent is typically used to determine if the target nerve is a candidate for neurotomy by RF ablation. If the nerve block via the pharmacological agent provides temporary pain relief to the patient, it is assumed that the target nerve is at least one source of the patient's pain, and an additional procedure will be scheduled for RF ablation of the same target nerve to provide the patient with long-term relief. When an initial RF ablation procedure is successful, the patient often returns for one or more additional RF ablation procedures months later as the effect of RF ablation can wear off due to regrowth of the target nerve.

The goal during each of the nerve blocking and RF ablation procedures is to deliver the pharmacological agent or RF energy to the exact same macroscopic site at the target nerve. The proper placement of the needle tip or probe tip next to the target nerve is essential for these procedures to be successful. In practice, however, it is difficult to exactly replicate the needle and probe placement from one procedure to the next because the target nerve cannot be directly visualized using presently available clinical methods. Thus, the physician must rely on images of bony landmarks to make an educated estimate of the true three-dimensional anatomical position of the target nerve. While various modalities such as fluoroscopy and ultrasound can be used to visualize bony landmarks during needle and probe placement, accurate needle and probe placement is a difficult art that requires skilled hands and years of training.

Therefore, despite the efforts of medical professionals to perform RF ablation on a target nerve previously identified via a nerve block with a pharmacological agent, some patients who experienced pain relief during the initial diagnostic nerve blocking procedure do not experience the same pain relief from the subsequent RF ablation procedure. Likewise, additional RF ablation procedures necessitated by recovery or regrowth of the target nerve often fail to provide the same pain relief as the first RF ablation procedure. One reason that such RF ablation procedures fail to provide pain relief is that the needle or probe is not placed in the same anatomical location that provided pain relief previously.

Therefore, a need exists for a system and method of treating pain where the tip of the RF ablation probe can be precisely placed in the same location as the needle tip during the initial diagnostic nerve block that was successful in providing temporary pain relief to the patient, or in a previous RF ablation procedure that was successful in providing pain relief to the patient. If the precise needle or probe location that facilitated previous pain relief was known, the medical professional could deliver energy to that exact location where the previous treatment was proven effective to ensure the success of any future RF ablation procedures.

EP 1 600 113 A3 discloses a prior art system having the features of the preamble of claim 1. Further prior disclosures that may be useful in understanding the present invention are provided in US 2009/024124 A1, US 2016/242661 A1, US 2014/135661 A1.

### Summary

In accordance with an aspect of the present invention, a system for improving location accuracy of a radiofrequency ablation procedure performed on a target nerve of a patient is provided as claimed in claim 1.

In one particular embodiment, the system can also include an obturator for facilitating insertion of the introducer into the patient.

In another embodiment, the target nerve can be a spinal nerve.

In another embodiment, the target nerve can be a peripheral nerve.

In still another embodiment, the fiducial marker can be sonopaque, radiopaque, or fluorescent.

In yet another embodiment, the introducer can deliver the pharmacological agent to the target nerve.

In another embodiment, the fiducial marker can be stored in the introducer until it is delivered to the target nerve.

In still another embodiment, the fiducial marker can be formed from a solid material.

In yet another embodiment, the fiducial marker can include one or more appendages, where the appendages anchor the marker to the target nerve or tissue adjacent the target nerve.

In one particular embodiment, the fiducial marker can be non-degradable. For example, the fiducial marker can be a metal.

In another embodiment, the fiducial marker can be degradable.

In still another embodiment, the pharmacological agent can be stored in a syringe, where the introducer receives the syringe to facilitate delivery of the pharmacological agent to the target nerve.

Other features and aspects of the present invention are discussed in greater detail below.

### Brief Description of the Drawings

A full and enabling disclosure of the present invention, including the best mode thereof to one skilled in the art, is set forth more particularly in the remainder of the specification, including reference to the accompanying figures, in which:
FIG. 1 is a block diagram of one method for improving location accuracy of a radiofrequency ablation procedure performed on a patient, outside the wording of the claims;
FIG. 2 is a perspective view of a system for improving location accuracy of a radiofrequency ablation procedure performed on a patient as contemplated by the present invention;
FIG. 3 is a cross-sectional view of one fiducial marker contemplated for use in the present invention;
FIG. 4 is a cross-sectional view of one more fiducial marker contemplated for use in the present invention;
FIG. 5 is a cross-sectional view of another fiducial marker contemplated for use in the present invention; and
FIG. 6 is a cross-sectional view of an additional fiducial marker contemplated for use in the present invention.

Repeat use of reference characters in the present specification and drawings is intended to represent the same or analogous features or elements of the present invention.

### Detailed Description

It is to be understood by one of ordinary skill in the art that the present discussion is a description of exemplary embodiments only, and is not intended as limiting the broader aspects of the present invention.

Generally speaking, the present disclosure is directed to a method (not claimed) and system for improving location accuracy of a radiofrequency ablation procedure performed on a patient. The focus of the present invention is to deliver radiofrequency energy during an ablation procedure to the exact same macroscopic site at which a temporary nerve block was performed after verifying that the temporary nerve block relieved the pain experienced by the patient.

The method includes a) identifying a target nerve to be ablated, where the target nerve is suspected of being a source of pain; b) delivering a pharmacological agent to the target nerve to temporarily block nerve signal transmission along the target nerve; c) verifying the target nerve is the source of pain if the temporary block reduces a level of pain experienced by the patient; d) placing a fiducial marker adjacent the target nerve if it is verified that the temporary block reduces the level of pain experienced by the patient; e) locating the fiducial marker; and f) delivering radiofrequency energy to an area of tissue adjacent the fiducial marker via a probe, where the area of tissue corresponds with the target nerve and the radiofrequency energy is applied at a level sufficient to ablate the target nerve.

Meanwhile, the system includes a probe having an outer diameter, a proximal end, and a distal end, the probe comprising an electrically insulated portion located at the proximal end and a conductive portion for delivering energy to a target nerve located at the distal end; an introducer for facilitating insertion of the distal end of the probe into the patient adjacent the target nerve, the introducer having an inner diameter, a proximal end, and a distal end, where the exposed conductive portion of the probe extends past the distal end during energy delivery to the target nerve, where the outer diameter of the probe and the inner diameter of the introducer define a lumen; a pharmacological agent supplied in an amount sufficient to block nerve signal transmission along the target nerve; and a fiducial marker configured for placement adjacent the target nerve. As used herein, the terms "distal" and "proximal" are defined with respect to the user/medical professional and when the device is in use. That is, the term "distal" refers to the part or portion further away from the user/medical professional and closest to the treatment site, while the term "proximal" refers to the part or portion closer to the user/medical professional and farthest from the treatment site when the device is in use.

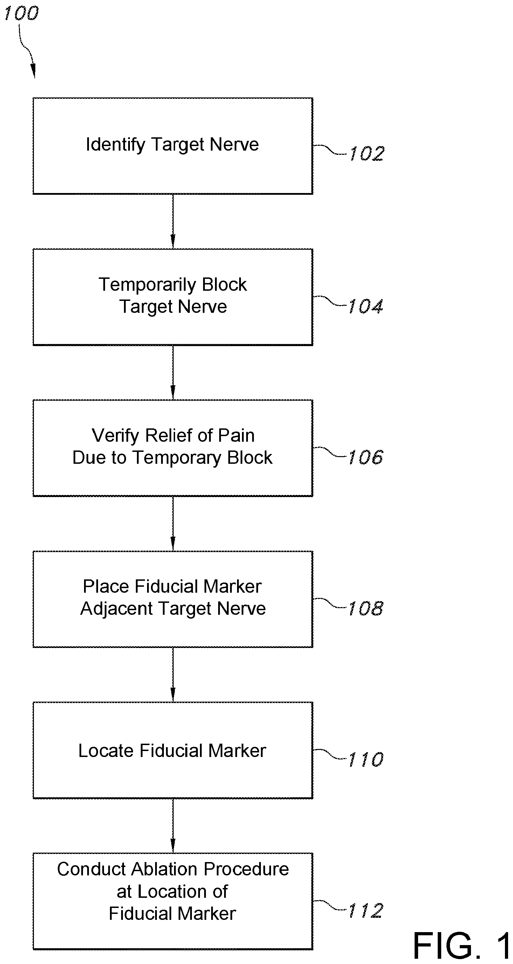

Referring now to the figures, and beginning with FIG. 1, various features of the method and system for improving the location accuracy of a radiofrequency ablation procedure performed on a patient are discussed in more detail. As shown in FIG. 1, the method 100 (outside the wording of the claims) includes identifying the target nerve 102, where the target nerve is suspected of being a source of pain. The target nerve can be a spinal nerve such as those nerves comprising motor, sensory and autonomic functions originating from the spinal cord in the body at the level of cranial, thoracic, lumbar, sacral or coccygeal regions of the spine. Additionally, peripheral nerves that innervate the limbs and distal digits may represent target nerves. The method then involves temporarily blocking nerve signal transmission along the target nerve 104. The nerve can be temporarily blocked with a pharmacological agent such as an anesthetic drug. The method then involves verifying relief of pain due to the temporary block 106, where it can be verified that the target nerve is the source of pain if the temporary block relieves or reduces the level of pain experienced by the patient. If relief of pain is achieved, indicating that the target nerve has been correctly identified and located, then the method further includes placing a fiducial marker adjacent the target nerve 108. Then, when the patient is ready for a more permanent or long lasting nerve block, such as one or more nerve blocks via RF ablation, the method further includes locating the fiducial marker 110, where the fiducial marker can be located via ultrasonography, radiography, fluoroscopy, etc. The method then includes conducting the RF ablation procedure at the location of the fiducial marker 112. By virtue of its location adjacent the target nerve, the fiducial marker provides guidance to the medical professional conducting the ablation procedure so that the target nerve can be accurately identified, resulting in the ablation of the correct target nerve at a precise location.

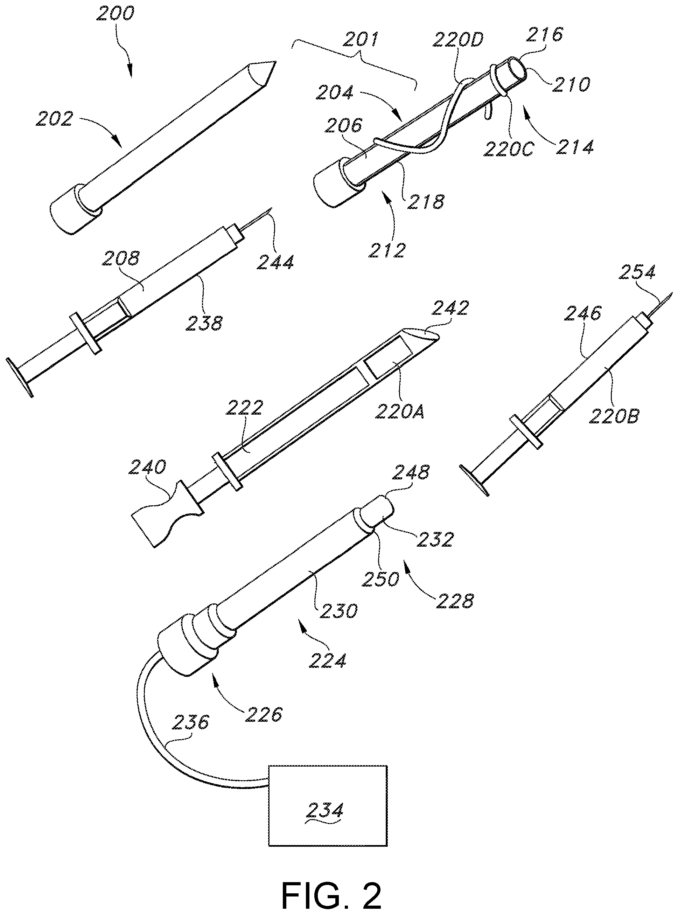

Referring now to FIG. 2, the nerve ablation system 200 that can be used to carry out the method 100 described above is discussed in more detail. First, the system 200 includes an introducer apparatus 201 that includes an introducer 204 and may also include an obturator 202 . The introducer apparatus 201 aids in the delivery of the pharmacological agent 208 for the temporary nerve block as well as in the insertion of a fiducial marker 220 and an RF ablation probe 224 into a patient's body. For example, the obturator 202 and the introducer 204 may be substantially stiff or rigid, such that the introducer apparatus 201 may assist in piercing the skin or other body tissues. In addition, the obturator 202 may be structured to cooperatively engage the introducer 204. In other words, the obturator 202 may be sized to fit within a lumen 206 of the introducer 204 as defined by the inner diameter 210 of the introducer, where the obturator 202 can include means for securing the obturator 202 to the introducer 204. In one embodiment, when the obturator 202 is fully disposed within the introducer 204, the obturator 202 sufficiently occludes the lumen 206 of the introducer 204 such that tissue is prevented from entering the lumen 206 when the introducer apparatus 201 is inserted into the body. In some embodiments the distal end of the obturator 202 may be sharp or pointed. In these embodiments, the distal end of the obturator 202 may be conical, beveled, or, more specifically, tri-beveled. The lengths of the obturator 202 and the introducer 204 may vary depending on the application. In one embodiment, the introducer 204 may be sized such that its distal end 214 can reach the target nerve tissue within the body while the proximal end 212 remains outside of the body. In some embodiments, such as when the target nerve is a spinal nerve, the introducer 204 can be between about 5.5 inches (13.97 cm) and about 7.5 inches (19.05 cm) in length, and obturator 606 may be between about 5.5 inches (13.97 cm) and about 7.5 inches (19.05 cm) in length. More specifically, the introducer 204 may be about 6.4 inches (16.26 cm) in length, and the obturator 202 may be about 6.6 inches (16.76 cm) in length. In other embodiments, such as when the nerve is a peripheral nerve, the introducer 204 can be from about 0.5 inches (1.27 cm) to about 2 inches (5.08 cm), such as from about 0.75 inches (1.91 cm) to about 1.75 inches (4.45 cm), such as from about 1 inch (2.54 cm) to about 1.5 inches (3.81 cm). The obturator 202 may be slightly longer than the introducer 204, so that the distal end of the obturator 202 may protrude from the introducer 204 when fully disposed. The lumen 206 of the
introducer 204 can also be sized to accommodate the diameter of the RF ablation probe 224, while remaining as small as possible in order to limit the invasiveness of the procedure. In a specific embodiment, the proximal ends of the introducer 204 and the obturator 202 are structured to be locked together with a hub or lock.

In one embodiment, the obturator 202 and the introducer 204 can be made from stainless steel. In other embodiments, the obturator 202, the introducer 204, or both may be made from other materials, such as nickel-titanium alloys, for example. Furthermore, in some embodiments, the obturator 202 can include a means for connecting the obturator 202 to an energy source or generator 234, for example a wire or cable. In such embodiments, the obturator 202 may be operable to measure the impedance of tissue as the introducer apparatus 201 is inserted into the patient's body. In addition or alternatively, the obturator 202 may be operable to deliver stimulation energy to a target nerve.

The system also includes a pharmacological agent 208. The pharmacological agent 208 can be delivered to the target nerve in order to temporarily block nerve signal transmission along the target nerve. By temporarily blocking nerve signal transmission along the target nerve, the temporary nerve block allows a medical professional the ability to verify that the correct nerve (i.e., the nerve causing pain for the patient) has been identified if the pain experienced by the patient is reduced or eliminated as a result of the temporary nerve block and delivery of the pharmacological agent 208 to the patient. The pharmacological agent 208 can be delivered to the target nerve by any suitable means. For instance, in one particular embodiment, the pharmacological agent 208 is stored in a syringe 238 having a needle tip 244. After the obturator 202 has been removed from the lumen 206 of the introducer 204, the syringe 238 can be inserted into the lumen 206 of the introducer 204 so that the needle tip 244 of the syringe 238 is positioned towards the distal end 214 of the introducer 204, which is adjacent the target nerve (i.e., the nerve suspected of being a source of pain). After the pharmacological agent 208 has been delivered to the target nerve in an amount sufficient to temporarily block nerve signal transmission at the target nerve, the syringe 238 can be removed. Although a syringe 238 is used in the discussion set forth above, it is to be understood that the pharmacological agent 208 can be delivered using a catheter, an IV bag, a pump, or any other suitable fluid delivery mechanism.

If delivery of the pharmacological agent 208 is successful in temporarily blocking nerve signal transmission along the target nerve, then the system 200 further includes a fiducial marker 220 to mark the target nerve so that a more permanent nerve block can be precisely and accurately applied to the target nerve to reduce or eliminate the level of pain experienced by the patient. The fiducial marker 220 can improve the accuracy of any subsequent nerve blocks that may take place minutes, hours, days, weeks, or even months after the initial procedure where the pharmacological agent 208 is delivered at one or more locations until the target nerve associated with the patient's pain is correctly identified. The fiducial marker 220 is delivered to the target nerve site (e.g., the site where the delivery of the pharmacological agent 208 reduced the patient's pain) from the introducer 204 and is disposed on an outer surface 218 of the introducer 204. In some embodiments, the fiducial marker 220 can be delivered to the target nerve through the introducer 204 without having to replace or remove the introducer 204, where the distal end 214 of the introducer 204 is positioned adjacent the target nerve. For instance, the fiducial marker 220 can be a fiducial marker 220A formed from a solid material. The solid material can be a metal, a polymer, a ceramic, or any other material that can be visualized using ultrasonography, radiography, or fluoroscopy, where the solid material may include an additive, such as a fluorescent material, to enable the material to be visualized. The solid material can be degradable or it can be non-degradable such that it can be removed during a subsequent procedure. If the fiducial marker 220A includes a metal, the metal can be gold, silver, titanium, platinum, or a combination thereof. If the fiducial marker 220A includes a non-metallic material, the material can be a degradable peptide polymer such as polylactic acid, poly(lactic-co-glycolic) acid and poly(caprolactone) or a non-peptide polymer and non-biodegradable polymer such as silicone, polyethylene, polyurethane and polypropylene. The fiducial marker can also include a proteinaceous biological material such as fibrin or collagen, a polysaccharide material such as chitosan, a glycosaminoglycan, or hyaluronic acid, or a ceramic such as a ceramic that includes hydroxyapatite. In addition, it is to be understood that any of the aforementioned materials can be used in any combination to form the fiducial marker 220A.

Regardless of the particular solid material utilized for the fiducial marker 220A, in an example not forming part of the invention, the fiducial marker 220A can be delivered from the introducer 204 to the target nerve via a deployment needle 240. The fiducial marker 220A can be stored at an open end 242 of the deployment needle 240, where the deployment needle 240 can be positioned inside lumen 206 the introducer 204 such that the open end 242 is adjacent the distal end 214 of the introducer 204 when delivery of the fiducial marker 220A to the target nerve or an area adjacent the target nerve is desired. A rod 222 disposed within the deployment needle 240 can then be manipulated to deploy the fiducial marker 220A from the open end 242 of the deployment needle 240 to the appropriate location adjacent the target nerve to mark the target nerve so that it can be accurately located during later procedures (e.g., RF ablation). The rod 222 can be formed of any material that is sufficiently rigid to allow for manipulation of the fiducial marker 220A into the desired location, such as a metal or a rigid polymer.

In another example outside the wording of the claims in which the fiducial marker 220 can be delivered to the target nerve through the introducer 204, where the distal end 214 of the introducer 204 is positioned adjacent the target nerve, the fiducial marker 220 can be a liquid fiducial marker 220B that hardens once it is delivered to the target nerve. For example, the liquid fiducial marker 220B can be a liquid polymer that hardens after it is injected or otherwise placed adjacent the target nerve. The liquid fiducial marker 220B can be stored in a syringe 246 having a needle tip 254, where the syringe 246 fits within the lumen 206 of the introducer 206 in order to deliver the liquid fiducial marker 220B to the target nerve through the needle tip 254. For instance, the liquid fiducial marker 220B can be a hydrogel and/or can be degradable. An example of a biodegradable hydrogel fiducial marker that can be used is described in U.S. Patent No. 7,329,414 to Fisher, et al. Specifically, the liquid fiducial marker 220B can be a hydrogel embedded with iodine, radium, beryllium, or other radiopaque contrast agent. Another example of a liquid fiducial marker 220B that is contemplated is an injectable radiopaque hydrogel, such as the TraceIT^{®} fiducial marker available from Augmenix, Inc., Waltham, MA, which includes iodinated polyethylene glycol hydrogel particles. Such a marker is visible for about 3 months, after which the particles liquefy and are absorbed and cleared in the patient's urine.

In other embodiments, the fiducial marker 220 can be formed from a solid material such as those discussed above and, according to an embodiment of the invention, is disposed on an outer surface 218 of the introducer 204 without having to replace or remove the introducer 204, where the distal end 214 of the introducer 204 is positioned adjacent the target nerve. For example, as shown in FIG. 2, the fiducial marker 220D can wrap around the outer diameter 216 of the introducer 204, and as the distal end 214 of the introducer 204 comes into contact with the target nerve, the fiducial marker 220D can "grasp" onto the target nerve and remain in place to mark the target nerve after removal of the introducer 204. The fiducial marker 220D can have a helical shape as shown, although any other suitable shape can also be used. For instance, the fiducial marker 220D can be c-shaped, ring-shaped, or linear such that the fiducial marker 220D points towards the target nerve at the site of drug or energy delivery.

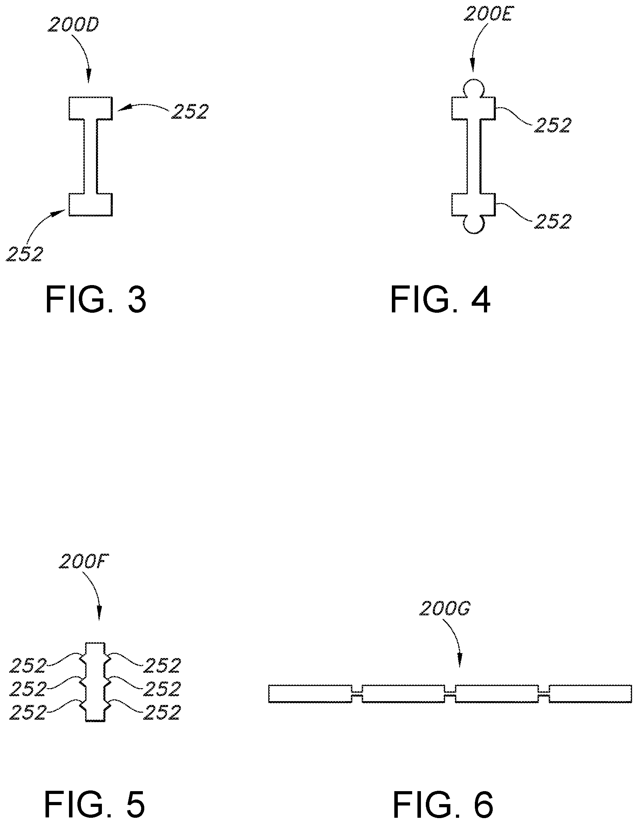

Referring now to FIGs. 3, 4, and 5, the present invention also contemplates fiducial markers 220D, 220E, and 220F, respectively, that include appendages 252. The appendages 252 help to anchor the fiducial markers 220D, 220E, and 200F in place adjacent the target nerve and prevent migration of the fiducial markers so that the target nerve can be accurately and precisely identified for later procedures (e.g., one or more RF ablation procedures) after the temporary nerve block. Although any suitable shape or design can be used for the appendages 252, FIG. 3 contemplates a fiducial marker 220D that has a barbell-like shape, where the ends of the barbell can be wedged into the target nerve or tissue adjacent the target nerve. Fiducial marker 220E of FIG. 4 has a similar barbell-like shape with the addition of semicircular appendages that further help to anchor the fiducial marker 220E in place. Meanwhile, fiducial marker 220F of FIG. 5 includes triangular-shaped appendages 252 along both sides of the length of the fiducial marker 220F, where the triangular-shaped appendages 252 can act as teeth to grasp the nerve tissue so that the fiducial marker 220F does not move and accurately mark the target nerve.

Still another design for a fiducial marker is show in FIG. 6, where the fiducial marker 220G has multiple "seeds" joined by thinner connection points. The seeds can provide for multiple reference points, and the thinner connection points provide the fiducial marker 220G with some degree of flexibility to manipulate the placement of the fiducial marker 220G adjacent the target nerve.

As mentioned above, in addition to an introducer apparatus 201, a pharmacological agent 208, and a fiducial marker 220, the system 200 contemplated by the present invention also includes an RF ablation probe 224. The probe 224 can be used to deliver RF energy to the target nerve, where the target nerve is accurately located by locating the fiducial marker 220 by ultrasonography, radiography, fluoroscopy, etc. Although FIG. 2 shows that the RF ablation device may be a probe 224 having an active probe tip 248, in other embodiments, the RF ablation device may be a cannula, a catheter, or any other elongate member capable of delivering energy to a target site within a patient's body. For the sake of clarity, the term "probe" is used throughout the specification to describe any such device. The probe 224 may be an elongate member and has a proximal end 226 and a distal end 228, where the term "distal" refers to the part or portion further away from the user and closest to the treatment site, while the term "proximal" refers to the part or portion closer to the user and farthest from the treatment site when the device is in use.

As shown in FIG. 2, the probe 224 includes an electrically insulated portion 230 and an electrically exposed conductive portion 232. The electrically exposed conductive portion 232 can also be referred to as an active electrode, and when the exposed conductive portion 232 is located at the distal end 228 of the probe 224, it may be referred to as an active tip 248. In general, the electrically insulated portion 230 may extend from the proximal end 226 of the probe 224 to a location adjacent the distal end 228 of the probe 224. The location to which electrically insulated portion 230 extends may depend on the application. Furthermore, the location to which electrically insulated portion 230 extends may not be fixed. In addition, in some embodiments, the probe 224 can include more than one electrically insulated portion 230 and/or more than one electrically exposed conductive portion 232.

In some embodiments, although not specifically shown, the proximal end 226 of the probe 224 can include a hub that can be structured to securely connect other devices such as introducers, connector cables, cannulae, tubes, or other hubs, for example, to the probe 224. For example, the probe 224 may be coupled to an energy source 234 and/or to a source of cooling via respective connecting means (for example, an electrical cable and/or flexible tubing) which may be associated with the hub. The hub may also serve as a handle or grip for the probe 224 and can serve as a locking mechanism to secure the probe 224 to the introducer 204. The hub may be manufactured from a number of different materials, including, but not limited to, plastics, polymers, metals, or combinations thereof. Furthermore, the hub may be attached to probe 224 by a number of different means. For example, in one embodiment, the hub may be made from polypropylene, and may be attached to the probe 224 by any suitable fitting such as a luer fitting. Although the hub can serve as a handle, it is also to be understood that a separate handle is also contemplated.

The size and shape of the probe 224 may vary depending on the application, and the invention is not limited in this regard. For example, in some embodiments, the transverse cross sectional shape of the probe 224 may be substantially circular. In other embodiments, the cross-sectional shape may be substantially polygonal, elliptical, or any other desired shape. In some embodiments, the length from the distal end 228 to the proximal end 226 of the probe 224 may be between about 5 centimeters (cm) and about 40 cm and the outer diameter 250 of the probe 224 may be between about 0.65 millimeters (mm) and about 2.00 mm (between about 20 AWG and about 12 AWG). In one specific example, the length of the probe 224 may be about 7.5 cm, the outer diameter may be about 1.5 mm, and the transverse cross-sectional shape may be substantially circular. Further, it is to be understood that the shape of the distal end 228 may vary depending on the application. Possible shapes include, but are not limited to, blunt, rounded, sharp, and beveled.

The probe 224 may be rigid or flexible and may be straight, bent or angled at one or more points along its length. As used herein, the term "bent" refers to any region of non-linearity or any deviation from a longitudinal axis, gradual or abrupt, and at any angle. In embodiments wherein the probe 224 is bent, the bend may be at various locations along the probe 224, for example, in the distal end 228. Furthermore, the bend may be of a variety of degrees and lengths. For example, the bend may traverse about 25° of a circle, and occur over a length of about 5 mm. In addition, the probe 224 can include a plurality of bends, which may or may not be in the same plane. For example, in some embodiments, the probe 224 may be bent such that it is helical or "corkscrew" shaped. In some embodiments, the probe 224 may be structured such that its shape may be modified by a user before or during the course of a procedure. More specifically, the shape of the distal end 228, for example, may be modified such that it may change from a straight to a bent configuration using an actuating mechanism. This may aid in accessing difficult to reach sites within the body and can be accomplished by a variety of means. For example, the probe 224 can include at least one active shape control mechanism, including but not limited to one or more pull-wires, a hydraulic or piezoelectric device, or another actuating mechanism.

In one embodiment, the electrically insulated portion 230 may be formed by coating a portion of the probe 224 with an electrically insulative coating, covering, or sheathing. In other words, the probe 224 can include electrically insulative material disposed on the surface of the elongate member. For example, in one embodiment, the probe 224 may be fabricated from a biocompatible metal or alloy, for example stainless steel, which may be overlaid in part by an insulating coating, for example polytetrafluoroethylene (PTFE). In other embodiments, the probe 224 can be fabricated from another metal, such as nitinol or titanium, and/or another electrically insulating material, including but not limited to polyethylene terephthalate (PET), may be disposed thereon. In other embodiments, other metals or electrically insulating materials may be used, and the invention is not limited in this regard. Furthermore, the insulating material may be semi-porous, to allow for some leakage of current through the insulating material. In some embodiments, the material may also be a thermal insulator as well. In still other embodiments, different insulating materials can be used for different portions of the probe 224. The insulating coating may be applied to a portion of the probe 224 by dip-coating, spraying or heat shrinking, for example. Meanwhile, the remaining uncoated portion of the distal end 228 of the probe 228 may serve as the electrically exposed conductive portion 232.

In another embodiment, the probe 224 can be fabricated from an insulative or non-conductive material and may be furnished with one or more externally applied electrodes (not shown). In such embodiments, the probe 224 can include one or more wires that may be attached to the electrode(s) at one end, and can run proximally along the length of the probe 224, such that a proximal portion of the wire(s) may be operatively connected to an energy source, thereby supplying energy to the electrodes. For example, the probe 224 can be fabricated from Radel^{™} plastic, and the externally applied electrodes can be fabricated from stainless steel.

In alternate embodiments, the probe 224 may be manufactured from a combination of materials. For example, the distal end 228 of the probe 224 can be made from a material such as nitinol, such that the shape of the distal end 228 may be altered, and the remainder the probe 224 may be made from stainless steel, such that the remainder of the probe 224 may be substantially fixed.

In some embodiments, the probe 224 may be cooled. In some specific embodiments, the probe 224 may be cooled by the internal circulation of a cooling fluid. Such a configuration, whereby a cooling medium does not exit from a distal end 228 of the probe 224, may be referred to as an internally-cooled probe. The cooling fluid may be any fluid suitable for removing heat from probe 224 during surgery, such as water. Other examples of cooling fluid include, but are not limited to, liquid nitrogen and saline. Furthermore, the cooling fluid may be at any temperature suitable for removing heat from the probe during surgery, for example between about 0°C and about 25°C. More specifically, the temperature of the fluid may be at about room temperature (21°C), about 4°C, or about 0°C, depending on the application.

In addition, the cooling fluid may be delivered or circulated at a wide range of flow-rates, and the invention is not limited in this regard. An appropriate flow-rate may be determined or calculated based on a number of factors, including the conductivity and heat capacity of the probe 224, the cooling fluid and/or the tissue, the internal structure of the probe 224, and the desired temperature of the distal end 228 of the probe 224, among other factors. In some embodiments, the cooling fluid may be delivered at flow ranging from about 10 milliliters/minute (ml/min) to about about 30 ml/min.

Means for cooling the probe 224 may include, but are not limited to, circulation of a cooling fluid, for example as described above, cooling by a thermoelectric circuit, or chemical cooling by an endothermic reaction. In some embodiments, the probe 224 may be cooled by a thermoelectric circuit. For example, the probe 224 may partially or fully house a circuit comprising two dissimilar metals or semiconductors, for example P- and N-doped bismuth-telluride, which are joined together at two junctions. When current passes through the circuit, heat may be transferred from one junction to the other. This phenomenon is known as the Peltier Effect. The junction where the heat is transferred from may be located in the distal end 228 of the probe 224, and the junction where the heat is transferred to may be located at a proximal end 226 of the probe 224 or externally to the probe 224. Energy may be provided to the circuit by an external energy source 234 (for example, the same energy source that delivers RF energy to the probe 224), an electrical generator or a battery, for example.

In an alternate embodiment, the probe 224 may be cooled chemically, where the probe 224 can include two or more separate materials that can be mixed when cooling is desired such that when mixed, an endothermic reaction or endothermic mixing occurs so that thermal energy will be absorbed, and the distal end 228 of the probe 224 will be cooled. The product(s) of the endothermic reaction or the resulting mixture may exit the probe 224 via the proximal end 226.

According to an embodiment of the invention, the introducer 204 is used to not only deliver the temporary nerve block (e.g., pharmacological agent 208) to the patient's body and/or facilitate insertion of the probe 224 as discussed above, but the introducer 204 is also used to deliver energy to the patient's body. The introducer 204 includes at least one electrically exposed conductive portion and at least one electrically insulated portion. In some embodiments, the body of the introducer 204 may be constructed from a conductive material, which is at least partially overlain with an insulating sheath or coating, defining the insulating region; however, in some embodiments, the introducer 204 may be constructed from an insulating material with one or more conductive bodies or electrodes applied externally. The distal end 214 of the introducer 204 may be pointed or sharp. For example, the distal end 214 of the introducer 204 can include a bevel. In one embodiment, the at least one electrically insulated portion may extend from the proximal end 212 of the introducer 204 to the distal end 214 of the introducer 204, such that the distal face of the introducer 204 includes at least one exposed conductive portion. In embodiments comprising a bevel, the at least one exposed conductive portion can include the bevel. In alternate embodiments, the exposed conductive portion may, alternatively or in addition, be located on a side of the introducer 204. In some embodiments, the electrical insulation may extend to the heel of the bevel of the introducer 204, while in others, the insulation may end further proximally along the introducer 204 .

In some embodiments, the introducer 204 is straight, whereas in some other embodiments the introducer 204 may be bent. For example, in some such embodiments, the introducer may have about a 5° to about a 20° bend in the distal end 214 of the introducer 204. In some embodiments, the introducer 204 may be between about 16 and about 18 AWG, between about 75 and about 150 mm in length, with the electrically exposed conductive portion about 2 mm to 6 mm in length. In these examples, the probe 224 may be structured to be disposed within the lumen 206 of the introducer 204 and to be in electrical contact with the introducer 204 when fully disposed within the introducer 204, which is outside the wording of the claims.

The probe 224 can include an electrically conductive elongated shaft, a connecting means for connecting to an energy source, and a connecting means for connecting to a cooling supply, for example as described herein above. Thus, when energy is supplied by an energy source to the probe 224, the energy flows along a conductive portion of the introducer 204 and is delivered to the target treatment site, traveling through the tissue or body to a reference or return electrode. In such the shaft of the probe 224 may be electrically conductive and exposed along substantially the entire length of the probe 224. In other words, a probe 224 used in such an example in conjunction with an introducer 204 may not include an electrically insulative coating 230 as described above, but this falls outside the wording of the claims.

In some examples outside the wording of the claims, the distal end 228 of the probe 224 may be substantially flush with the distal end 214 of the introducer 204 when fully disposed in the introducer 204. In other embodiments, the distal end 228 of the probe 224 may extend distally from the distal end 214 of the introducer 204 when fully disposed in the introducer 204. In other examples outside the wording of the claims, the distal end 228 of the probe 224 may be recessed proximally from the distal end 214 of the introducer 204 when fully disposed in the introducer 204. As used herein, the phrase "fully disposed" refers to a first member being substantially fully received within a second member such that, under normal use, it is not intended to be inserted into the second member any further.

The probe 224 and the introducer 204 may be structured such that when the probe 224 is disposed within the introducer 204, at least a portion of the probe 224 is in electrical and/or thermal contact with at least a portion of the introducer 204, such that thermal and/or electrical energy may be delivered from the probe 224 to the introducer 204. This may be accomplished by flushing the introducer 204 with a liquid such as saline prior to inserting the probe 224, such that a layer of liquid remains between at least a portion of the probe 224 and the introducer 204. The saline may then serve to conduct electricity and/or heat between the probe 224 and the introducer 204. Alternatively, the probe 224 and introducer 204 may be structured such that they are in physical contact when the probe 224 is fully disposed within the introducer 204, thereby also being in electrical and thermal contact. In a further embodiment, a portion of the probe 224 may be in thermal contact with the conductive portion of the introducer 204. This may be beneficial in that the cooling of the probe 224 would allow for the conductive portion of the introducer 204 to be cooled. The probe 224 may be cooled by a variety of methods, as described above.

In some embodiments, the probe 224 may be structured to be operatively connected to an energy source 234, for example a generator 234. The connecting means 236 for connecting the probe 224 to the generator 234 can include any component, device, or apparatus operable to make one or more electrical connections, for example an insulated wire or cable. In one embodiment, the connecting means 236 can include an electrical cable terminating at the proximal end 226 of the probe 224. The connector may be operable to couple to the energy source 234 directly or indirectly, for example via an intermediate cable. At least one wire or other electrical conductor associated with the cable 236 may be coupled to a conductive portion of the probe 224, for example by a crimp or solder connection, in order to supply energy from the energy source 234 to the probe 224. In one specific embodiment, a 4-pin medical connector may be used to connect the cable 236 to an intermediate cable (not shown), which may be further attached to a 14-pin connector capable of being automatically identified when connected to the energy source/generator 234.

The energy source/generator 234 may produce various types of energy, for example microwave, ultrasonic, optical, or radio-frequency electrical energy. In some embodiments, generator 234 may produce radiofrequency electrical current, having a frequency of between about 10 kHz and about 1000 kHz, at a power of between about 1 Watts and about 50 Watts. In some embodiments, the generator 234 can include a display means incorporated therein. The display means may be operable to display various aspects of a treatment procedure, including but not limited to any parameters that are relevant to a treatment procedure, such as temperature, power or impedance, and errors or warnings related to a treatment procedure. Alternatively, the generator 234 can include means for transmitting a signal to an external display. In one embodiment, the generator 234 may be operable to communicate with one or more devices, for example with one or more probes 224 and/or one or more sources of cooling (not shown). Such communication may be unidirectional or bidirectional depending on the devices used and the procedure performed. Further details regarding embodiments of energy sources are disclosed in U.S. Patent No. 8,882,755 to Leung, et al. and U.S. Patent No. 7,258,688 to Shah, et al.

As an example of communication between the generator 234 and other devices in a system of the present invention, the generator 234 may receive temperature measurements from one or more temperature sensing devices (not shown). Based on the temperature measurements, the generator 234 may perform some action, such as modulating the power that is sent to the probe(s). For example, power to the probe(s) could be increased when a temperature measurement is low or decreased when a measurement is high, relative to a predefined threshold level. If more than one probe is used, the generator 234 may be operable to independently control the power sent to each probe depending on the individual temperature measurements received from the temperature sensing devices associated with each probe. In some cases, the generator 234 may terminate power to one or more probe(s) 224. Thus, in some embodiments, the generator 234 may receive a signal (e.g., temperature measurement) from one or more probe(s), determine the appropriate action, and send a signal (e.g., decreased or increased power) back to one or more probe(s).

Alternatively, if one or more cooling means (i.e., sources of cooling) includes one or more pumps (not shown), the one or more pumps may communicate a cooling fluid flow rate to the generator 234 and may receive communications from the generator 234 instructing the pump(s) to modulate this flow rate depending, for example, on temperature measurements received by the generator 234. In some embodiments, the pump(s) may respond to the generator 234 by changing the flow rate or by turning off for a period of time. The pumps may be turned off in order to allow the temperature of the tissue surrounding the probe 224 to reach equilibrium, thereby allowing a more precise determination of the surrounding tissue temperature to be made. In addition, when using more than one probe 224, in embodiments where the generator 234 does not control each of the probes 224 independently, the average temperature or a maximum temperature in the temperature sensing devices associated with probe(s) 224 may be used to control the cooling means.

Systems of the present invention include a probe 224; in some embodiments, systems of the present invention can include a plurality of probes, for example, two probes. The system may be operated, for example, in a monopolar mode, a bipolar mode, or a multiphasic/multi-polar mode. When operated in a monopolar mode, any number of probes may be used, and the system may further include a dispersive return electrode. The dispersive return electrode may be, for example, a grounding pad for attaching to the patient's skin, or may be a substantially large electrode that is integral with the probe 224. When the system is operated in a bipolar mode, any number of probes, for example, two probes, may be used, and current may travel between the probes. Alternatively, when one probe 224 is used, current may travel between a conductive portion 232 and a second electrically conductive and exposed portion on the probe 224. For example, the probe 224 can include a second electrically conductive and exposed portion in the form of a ring that is disposed around the probe 224 at a location proximal to the conductive portion 232. The conductive portion 232 and the second electrically conductive and exposed portion may be electrically isolated from each other, and the probe 2224 can include means for operatively connecting the second electrically conductive and exposed portion to a source of energy which is at a different electrical potential than the conductive portion 232, or to a circuit ground.

The operation of the system may be manually controlled by a user, or may be automatically controlled based on certain parameters, for example, based on a measurement of a property of a component of is the system itself or of a property of the tissue being treated. When more than one probe is used, means of controlling the operation of the system may be configured to independently control each probe such that, for example, current flow to any of the probes may be independently adjustable. In addition, a flow of cooling may be controlled independently to each probe. Thus, if one probe is found to be at a higher temperature relative to another probe or probes, flow of cooling to that probe may be increased and/or current flow to that probe may be decreased. Similarly, if one probe is found to be at a lower temperature relative to another probe or probes, flow of cooling to that probe may be decreased and/or current flow to the probe may be increased. In embodiments of a system having automatic control, the system can include a controller operable to control one or more devices based on specified criteria. Further details regarding automatic or manual control of the system are provided in U.S. Patent No. 8,882,755 to Leung, et al.

In general, examples of a method of the present disclosure involve using a treatment device, for example an electrosurgical device including a probe, in a particular region of a patient's body to form a lesion of sufficient size and suitable geometry to effectively block nerve signal transmission along the target nerve (e.g., a spinal nerve) to reduce a level of pain experienced by a patient. For example, in one broad aspect, a method is provided for creating a lesion at a target site within a body of a human or animal using an electrosurgical device having a longitudinal axis in order to ablate the target nerve. The ablation procedure can include the steps of: inserting the probe into the body via an introducer; delivering a liquid (e.g., therapeutic agent, saline, etc.) from a liquid introduction apparatus (e.g., syringe, IV bag, etc.) into a port and through a distal end of the introducer; optionally removing the liquid introduction apparatus to provide the electrosurgical device with a vent; and delivering energy from an energy source through a distal end of the introducer to the target nerve for creating the lesion at the target nerve, wherein the probe is a cooled probe.

The desired size and geometry of the lesion may depend on the specific anatomy and tissue being targeted and may be affected by several parameters as described herein, including but not limited to the geometry of the treatment device and the amount of cooling delivered to the treatment device. Thus, steps are provided for creating a lesion with desired characteristics during the course of an electrosurgical procedure. The lesion may function to inhibit neural activity, for example nociception. Method examples of the present disclosure can generally include one or more of the steps of: determining a desired lesion shape, size, and location; selecting an electrosurgical instrument or device, for example a probe, and energy delivery parameters, for example voltage, based on the desired lesion shape, size, and location; inserting the electrosurgical instrument or device into a patient's body; positioning the electrosurgical instrument or device at a target site; delivering energy, for example radiofrequency current, through the electrosurgical instrument or device to the target site to form a lesion; and applying cooling to the electrosurgical instrument or device. As will presently be discussed, examples of the method aspect of the present disclosure may be useful, for example, to allow for more straightforward device placement during electrosurgical procedures than is presently possible.

In one example of the method aspect of the present disclosure, the step of inserting an electrosurgical instrument or device can include inserting a probe percutaneously into a patient's body, and the step of positioning an electrosurgical instrument or device can include advancing the electrosurgical instrument or device until the active electrode is at or in the vicinity of a target nerve treatment site. The step of inserting a probe is preceded by one or more additional steps that can include, for example, inserting an introducer apparatus into the body in the vicinity of the target treatment site, measuring one or more properties of a device or of tissue at or near the target nerve, inserting or removing material at or near the target nerve, delivering a pharmacological agent to the target nerve to create a temporary nerve block, placing a fiducial marker adjacent the target nerve, locating the fiducial marker to facilitate insertion of the probe and delivery of the energy at a precise and accurate location adjacent the target nerve, and/or performing another treatment procedure at or near the target treatment site.

As described above, the pharmacological agent 208, the fiducial marker 220, and the probe 224 are used in conjunction with an introducer apparatus 201, which includes an introducer 204 and can include an obturator 202. In use, the obturator 202 may be initially disposed within a lumen 206 of the introducer 204 to facilitate insertion of the introducer apparatus 201 to the target nerve site. Once the introducer apparatus 201 has been properly positioned, the obturator 202 may be removed and replaced within the introducer lumen 206 by the syringe 238 or other apparatus for delivering the pharmacological agent 208, by the syringe 246 for delivering the fiducial marker 220 to the target nerve, or by the probe 224. In some embodiments, the obturator 202 may be operable to measure the impedance of tissue as the introducer apparatus 201 is inserted into the patient's body, which may assist in positioning the introducer apparatus 201 at the target nerve. Alternatively or in addition, the obturator 202 may be operable to deliver stimulation energy to the target nerve.

In any event, when a medical professional believes that he or she has positioned the introducer 204 so that its distal end 214 is adjacent the target nerve, which is the nerve suspected of causing the patient pain, the pharmacological agent 208 can then be delivered through the introducer 204, such as via syringe 238, to temporarily block nerve signal transmission along the target nerve. If the patient indicates that his or her pain level has been reduced as a result of the temporary block, then verification is provided that the correct nerve has been identified. Thereafter, the target nerve can be marked or tagged with any of the fiducial markers 220A-220G as described above. In some instances, the introducer 204 can then be removed, such as when the more permanent nerve block (e.g., RF ablation) is scheduled to occur at a later time or date. Because of the use of the fiducial maker 220, the target nerve can be accurately identified when it is time to perform the RF ablation procedure. For example, ultrasonography, radiography, fluoroscopy, etc. are used to identify the fiducial marker 220 and thus the target nerve. Then, once the fiducial marker 222 is identified, the introducer apparatus 201 can be reinserted into the patient's body such that the distal end 216 of the introducer 204 is adjacent the fiducial marker 220 and target nerve. Then, the probe 224 is inserted into the lumen 206 of the introducer 204 in order to apply radiofrequency energy at a level sufficient to ablate the target nerve, thus providing the patient with longer lasting pain relief compared to the temporary block with the pharmacological agent.

More specifically, after delivery of the pharmacological agent to create a temporary nerve block to verify that the target nerve that is causing pain has been correctly identified, and after the fiducial marker has been placed at or adjacent the correctly identified target nerve, by delivery from the outer surface of the introducer, the introducer can be removed from the patient's body. Depending on the RF ablation treatment schedule, then the introducer can be reinserted at a later time (e.g., minutes, hours, days, weeks, or months later) via the obturator as described above, after which the probe can be inserted into the lumen of the introducer. The probe and the introducer are structured such that when the probe is fully disposed within the introducer, the distal
end of the probe protrudes or extends from the distal end of the introducer. For example, as described above, if the introducer includes an electrically conductive elongate member covered by electrically insulating material, with a distal portion that is electrically conductive and exposed, then the probe may be operable to deliver energy from an energy source to the conductive distal portion of the introducer. This delivery of energy may be facilitated by physical contact between the tip of the probe and the inner surface of the introducer. In such an example, outside the wording of the claims, the probe tip may be aligned with the distal end of the introducer and the length of the exposed conductive portion of the introducer will affect characteristics of the resulting lesion. Alternatively, in some embodiments, the introducer can include an electrically insulated elongate member not having a conductive and exposed distal portion. The distal end of the probe protrudes or extends from the distal end of the introducer and the distance that the probe tip extends may be altered by advancing or retracting the probe. The distance that the probe tip extends from the introducer will affect the formation of the lesion.

During the steps of inserting and positioning the probe, the probe may be inserted and positioned such that the distal end of the probe, comprising the active electrode, is the portion of the probe that is closest to the treatment site (e.g., the target nerve), where the target nerve is correctly identified by locating the fiducial marker via ultrasonography, radiography, fluoroscopy, etc. If the treatment site includes a surface, for example, the probe may be inserted and positioned substantially perpendicular or generally upstanding to the surface, for example at an angle between about 80° and about 100° relative to the surface. In other embodiments, the probe may be positioned at an angle between about 45° and 135° or, in alternate embodiments, between about 60° and 120°. The probe may be inserted and positioned such that the distal end of the probe is directly adjacent to, or in contact with the target treatment site, or may be inserted and positioned such that the distal end of the probe is proximal to the target site. For example, in one embodiment, a probe may be inserted and positioned using what may be described as a "perpendicular" or "gun-barrel" approach. In this embodiment, the probe may be directed to the target site such that its longitudinal axis is substantially perpendicular or generally upstanding to the line or plane formed by the target tissue or site. For example, if the target tissue is a nerve, the probe may be positioned such that the probe is substantially perpendicular or generally upstanding relative to the nerve. If the target tissue includes more than one neural structure, such as a nerve or nerve branch, the probe may be inserted and positioned such that it is substantially perpendicular or generally upstanding to a plane containing the neural structures. As will be described in more detail below, embodiments of the present invention may allow for the creation of a lesion that is located primarily distally with respect to the distal end of a probe, thus allowing a probe that has been inserted substantially perpendicularly or generally upstanding relative to a target nerve to effectively treat the target nerve by delivering energy to form a lesion distal to the probe.

In alternate embodiments, the probe may be inserted at various angles to the target nerve, depending on the procedure being performed and the anatomical structures involved. For example, in some embodiments, the probe may be inserted such that it is substantially parallel to a target nerve, for example at an angle of between about 0° and about 20°. In other embodiments, the probe may be inserted such that it is at an angle of between about 20° to about 70° to the target nerve. In general, embodiments of the present invention allow for various angles of approach by providing an apparatus and method of use thereof for creating a lesion of variable size and at various locations relative to the apparatus.

The step of inserting and positioning a probe may involve the insertion of a single probe to a location in the vicinity of a single target treatment site, the insertion of multiple probes in the vicinity of a single target treatment site, or the insertion of multiple probes to multiple locations in the vicinity of multiple target treatment sites. The probe or probes may be configured to deliver energy in a monopolar, bipolar or multi-polar configuration. If the probe or probes are configured to deliver energy in a monopolar configuration, the method of the current disclosure may also include a step of placing a reference electrode, such as a grounding pad, at another location on or in the body. The steps of inserting and positioning a probe may optionally be followed by any number of steps, for example prior to the commencement of the step of delivering energy including, but not limited to, one or more of: measuring one or more properties of a device or of tissue at or near the target treatment site; applying a stimulation signal to a tissue (for example, neural tissue) at or near the target treatment site; measuring the reaction to stimulation (for example, the somato-sensory evoked potential, or SSEP) of a tissue (for example, muscular or neural tissue) in response to the application of a stimulation signal at or near the target treatment site; inserting or removing material at or near the target treatment site; and performing another treatment procedure at or near the target treatment site. Further details regarding these steps may be found in U.S. Patent No. 8,882,755 to Leung, et al., U.S. Patent No. 7,819,869 to Godara, et al., U.S. Patent No. 8,951,249 to Godara, et al., U.S. Patent Application Publication No. 2006/0259026 to Godara, et al., and U.S. Patent No. 8,096,957 to Conquergood, et al. Following the performance of one or more of the above optional steps, one or more probes may be reinserted, moved, or otherwise repositioned and any optional steps may then be repeated.

The step of delivering energy to the target nerve, for example to create a lesion at the target nerve, may involve the creation of a lesion of a desired shape and at a desired location relative to the probe. As mentioned hereinabove, lesion shape and location may be affected by the length of the exposed distal end of the probe. The less of the probe that is exposed, the more distally, relative to the probe, the lesion will form. In addition, the shape of the lesion will be generally more spherical if less of the tip is exposed. For example, if the exposed length of the distal end is limited substantially to the distal-most hemisphere (i.e., the face) of the tip, then a substantially spherical lesion may form primarily distally with respect to the probe. Such a probe may be positioned such that the active electrode of the probe lies substantially proximal from the target site, for example a nerve. Energy may then be delivered to the probe such that a lesion may form substantially distal to the active electrode of the probe. Conversely, if more of the tip is exposed, then the lesion will appear more oblate and may form more radially (i.e. perpendicular to the longitudinal axis of the probe) around the distal end and the component of the lesion distal to the distal end will decrease.

The type, parameters, and properties of the energy delivered to the probe may vary depending on the application, and the invention is not limited in this regard. The energy may be one of various types of energy, for example electromagnetic, microwave, or thermal. In some embodiments, radiofrequency electrical current having a frequency of between about 10 kHz and about 1000 kHz, at a power of about 50 Watts, may be delivered to the probe.

In some examples of the method of the present disclosure, the step of delivering energy (e.g., radiofrequency (RF) energy) to the tissue may be preceded by, and/or done coincidently with, a step of applying cooling. Cooling may be used to reduce the temperature of the tissue in the vicinity of the site of energy delivery, allowing more energy to be applied without causing an increase to an unsafe temperature in local tissue. The application of more energy allows regions of tissue further away from the electrode(s) to reach a temperature at which a lesion can form, thus increasing the maximum size/volume of the lesion. Furthermore, depending on the structure of the probe, cooling may allow for a lesion to form at a position that is substantially distal to and, in some examples, spaced from the probe. Further details regarding cooled probes are disclosed in U.S. Patent Application Publication No. 2007/0156136 to Godara, et al. and U.S. Patent No. 7,819,869 to Godara, et al. Thus, cooling an electrosurgical probe may change the size, shape, and location of formation of a lesion. As noted above, the theory described herein regarding tissue heating and lesion formation is not intended to limit the present invention in any way.

Methods of the present disclosure may be used for various applications, including for the treatment of pain associated with many conditions. Examples of such conditions include, but are not limited to, Complex Regional Pain Syndrome (CRPS), Trigeminal Neuralgia, Joint Specific Peripheral Neuropathy, Facet Joint Pain, Intervertebral disc pain, Sacroiliac Joint Syndrome (SIJS), neuromas and Hypogastric or Pelvic Pain. In general, these conditions may be treated by affecting at least one target neural structure that may be associated with a patient's pain in accordance with method examples of the present disclosure. For example, in the case of trigeminal neuralgia, examples of the present disclosure may be used to form a lesion at the trigeminal nerve. Some examples of a method of the present disclosure may also be used to treat further sources of pain, as will be described in more detail below.

In one example, the patient may be placed in the prone position in preparation for the RF ablation procedure. The medical professional may optionally administer various treatments, such as anesthetics or antibiotics, for example. The medical professional may insert at least one probe, such as probe 224 described hereinabove, percutaneously towards the target nerve/fiducial marker site, using the fiducial marker 220 and one or more imaging techniques (e.g., ultrasonography, radiography, fluoroscopy, etc.) for accurate placement of the probe 224. The step of inserting at least one probe 224 can include the use of an introducer apparatus 201, as described above. Such an apparatus may be an introducer apparatus 201 that includes an introducer 204 and an obturator 202. The position of the probe 224 may be pre-determined based on the desired lesion size, shape, and location. When the introducer apparatus 201 has been positioned, the medical professional may withdraw the obturator 202 from the introducer 204, leaving the introducer 204 in place. Depending on the positioning of the introducer 204, the distal end 214 of the introducer 204 may now be touching the bone, or may be some distance proximal from the bony surface, for example about 3 mm away from the bone. The user may then insert a probe 224 into the lumen 206 of the introducer 204. The probe 224 may be operatively connected to a source of cooling fluid and may further be operatively connected to a source of energy, such as generator 234, in order to deliver energy to the target nerve, using the fiducial marker 220 as a guide for accurate placement of the probe 224.

As described above, depending on the configuration and positioning of the probe, as well as the degree of cooling, the lesion formed at the target nerve may be of a variety of shapes and sizes, as described above.

These and other modifications and variations of the present invention may be practiced by those of ordinary skill in the art, without departing from the scope of the present invention. In addition, it should be understood that aspects of the various embodiments may be interchanged both in whole and in part. Furthermore, those of ordinary skill in the art will appreciate that the foregoing description is by way of example only, and is not intended to limit the invention so further described in such appended claims.

## Claims

1. A system for improving location accuracy of a radiofrequency ablation procedure performed on a target nerve of a patient, the system (200) comprising:
a probe (224) having an outer diameter (250), a proximal end (226), and a distal end (228), the probe (224) comprising an electrically insulated portion (230) located at the proximal end (226) and a conductive portion (232) for delivering energy to a target nerve located at the distal end (228);
an introducer (204) for facilitating insertion of the distal end (228) of the probe (224) into the patient adjacent the target nerve, the introducer (204) having an inner diameter (210), a proximal end (212), and a distal end (214), wherein the exposed conductive portion (232) of the probe (224) is configured to extend past the distal end (214) of the introducer during energy delivery to the target nerve, wherein the inner diameter (210) of the introducer (204) defines a lumen (206);
a pharmacological agent (208) for blocking nerve signal transmission along the target nerve; and
a fiducial marker (220) configured for placement adjacent the target nerve, wherein the fiducial marker (220) is configured to be deployed from the introducer (204),
**characterized in that**:
the fiducial marker (220) is disposed on an outer surface (218) of the introducer (204).

2. The system of claim 1, further comprising an obturator (202) for facilitating insertion of the introducer (204) into the patient.

3. The system of claim 1 or 2, wherein the target nerve is a spinal nerve or a peripheral nerve.

4. The system of any preceding claim, wherein the fiducial marker (220) is sonopaque, radiopaque, or fluorescent.

5. The system of any preceding claim, wherein the introducer (204) is configured to deliver the pharmacological agent (208) to the target nerve.

6. The system of any preceding claim, wherein the introducer (204) is configured for storage of the fiducial marker (220) until it is delivered to the target nerve.

7. The system of any preceding claim, wherein the fiducial marker (220) is formed from a solid material.

8. The system any preceding claim, wherein the fiducial marker (220) includes one or more appendages (252), wherein the appendages (252) are configured to anchor the fiducial marker (220) to the target nerve or tissue adjacent the target nerve.

9. The system of any preceding claim, wherein the fiducial marker (220) is non-degradable.

10. The system of claim 9, wherein the fiducial marker (220) is a metal.

11. The system of any one of claims 1 to 8, wherein the fiducial marker (220) is degradable.

12. The system of any one of claims 1 to 8 further comprising a syringe (238), wherein the pharmacological agent (208) is configured for storage in the syringe (238), wherein the introducer (204) is configured to receive the syringe (238) to facilitate delivery of the pharmacological agent (208) to the target nerve.

## Patentansprüche

1. System zum Verbessern der Positionsgenauigkeit eines an einem Zielnerv eines Patienten durchgeführten Hochfrequenzablationsverfahrens, wobei das System (200) Folgendes umfasst:
eine Sonde (224), die einen Außendurchmesser (250), ein proximales Ende (226) und ein distales Ende (228) aufweist, wobei die Sonde (224) einen elektrisch isolierten Abschnitt (230) umfasst, der sich am proximalen Ende (226) befindet, und einen leitfähigen Abschnitt (232) zum Abgeben von Energie an einen Zielnerv, der sich am distalen Ende (228) befindet, umfasst;
eine Einführungsvorrichtung (204) zum Erleichtern des Einführens des distalen Endes (228) der Sonde (224) in den Patienten neben dem Zielnerv, wobei die Einführungsvorrichtung (204) einen Innendurchmesser (210), ein proximales Ende (212) und ein distales Ende (214) aufweist, wobei der freiliegende leitfähige Abschnitt (232) der Sonde (224) konfiguriert ist, um sich während der Energieabgabe an den Zielnerv über das distale Ende (214) der Einführungsvorrichtung hinaus zu erstrecken, wobei der Innendurchmesser (210) der Einführungsvorrichtung (204) ein Lumen (206) definiert;
ein pharmakologisches Mittel (208) zum Blockieren der Nervensignalübertragung entlang des Zielnervs; und
eine Bezugsmarkierung (220), die zum Platzieren neben dem Zielnerv konfiguriert ist, wobei die Bezugsmarkierung (220) konfiguriert ist, um von der Einführungsvorrichtung (204) eingesetzt zu werden,
**dadurch gekennzeichnet, dass**:
die Bezugsmarkierung (220) an einer Außenoberfläche (218) der Einführungsvorrichtung (204) angeordnet ist.

2. System nach Anspruch 1, das weiter einen Obturator (202) zum Erleichtern des Einsetzens der Einführungsvorrichtung (204) in den Patienten umfasst.

3. System nach Anspruch 1 oder 2, wobei der Zielnerv ein Spinalnerv oder ein peripherer Nerv ist.

4. System nach einem vorstehenden Anspruch, wobei die Bezugsmarkierung (220) tonundurchlässig, funkundurchlässig oder fluoreszierend ist.

5. System nach einem vorstehenden Anspruch, wobei die Einführungsvorrichtung (204) konfiguriert ist, um das pharmakologische Mittel (208) an den Zielnerv abzugeben.

6. System nach einem vorstehenden Anspruch, wobei die Einführungsvorrichtung (204) zum Aufbewahren der Bezugsmarkierung (220) konfiguriert ist, bis diese an den Zielnerv abgegeben wird.

7. System nach einem vorstehenden Anspruch, wobei die Bezugsmarkierung (220) aus einem festen Material gebildet ist.

8. System nach einem vorstehenden Anspruch, wobei die Bezugsmarkierung (220) einen oder mehrere Anhänge (252) beinhaltet, wobei die Anhänge (252) konfiguriert sind, um die Bezugsmarkierung (220) am Zielnerv oder am Gewebe neben dem Zielnerv zu verankern.

9. System nach einem vorstehenden Anspruch, wobei die Bezugsmarkierung (220) nicht abbaubar ist.

10. System nach Anspruch 9, wobei die Bezugsmarkierung (220) ein Metall ist.

11. System nach einem der Ansprüche 1 bis 8, wobei die Bezugsmarkierung (220) abbaubar ist.

12. System nach einem der Ansprüche 1 bis 8, das weiter eine Spritze (238) umfasst, wobei das pharmakologische Mittel (208) zum Aufbewahren in der Spritze (238) konfiguriert ist, wobei die Einführungsvorrichtung (204) konfiguriert ist, um die Spritze (238) aufzunehmen, um die Abgabe des pharmakologischen Mittels (208) an den Zielnerv zu erleichtern.

## Revendications

1. Système pour améliorer la précision de localisation d'une procédure d'ablation par radiofréquence effectuée sur un nerf cible d'un patient, le système (200) comprenant :
une sonde (224) présentant un diamètre externe (250), une extrémité proximale (226) et une extrémité distale (228), la sonde (224) comprenant une partie électriquement isolée (230) située au niveau de l'extrémité proximale (226) et une partie conductrice (232) servant à délivrer de l'énergie à un nerf cible située à l'extrémité distale (228) ;
un introducteur (204) pour faciliter l'insertion de l'extrémité distale (228) de la sonde (224) dans le patient à proximité du nerf cible, l'introducteur (204) présentant un diamètre interne (210), une extrémité proximale (212) et une extrémité distale (214), dans lequel la partie conductrice exposée (232) de la sonde (224) est configurée pour s'étendre au-delà de l'extrémité distale (214) de l'introducteur pendant la délivrance d'énergie au nerf cible, dans lequel le diamètre interne (210) de l'introducteur (204) définit une lumière (206) ;
un agent pharmacologique (208) pour bloquer la transmission du signal nerveux le long du nerf cible ; et
un marqueur de repère (220) configuré pour être placé à proximité du nerf cible, dans lequel le marqueur de repère (220) est configuré pour être déployé à partir de l'introducteur (204),
**caractérisé en ce que** :
le marqueur de repère (220) est disposé sur une surface externe (218) de l'introducteur (204).

2. Système selon la revendication 1, comprenant en outre un obturateur (202) pour faciliter l'insertion de l'introducteur (204) dans le patient.

3. Système selon la revendication 1 ou 2, dans lequel le nerf cible est un nerf spinal ou un nerf périphérique.

4. Système selon une quelconque revendication précédente, dans lequel le marqueur de repère (220) est sono-opaque, radio-opaque ou fluorescent.

5. Système selon une quelconque revendication précédente, dans lequel l'introducteur (204) est configuré pour délivrer l'agent pharmacologique (208) au nerf cible.

6. Système selon une quelconque revendication précédente, dans lequel l'introducteur (204) est configuré pour contenir le marqueur de repère (220) jusqu'à ce qu'il soit délivré au nerf cible.

7. Système selon une quelconque revendication précédente, dans lequel le marqueur de repère (220) est formé d'un matériau solide.

8. Système selon une quelconque revendication précédente, dans lequel le marqueur de repère (220) inclut un ou plusieurs appendices (252), dans lequel les appendices (252) sont configurés pour ancrer le marqueur de repère (220) au nerf cible ou à un tissu adjacent au nerf cible.

9. Système selon une quelconque revendication précédente, dans lequel le marqueur de repère (220) est non dégradable.

10. Système selon la revendication 9, dans lequel le marqueur de repère (220) est un métal.

11. Système selon l'une quelconque des revendications 1 à 8, dans lequel le marqueur de repère (220) est dégradable.

12. Système selon l'une quelconque des revendications 1 à 8, comprenant en outre une seringue (238), dans lequel l'agent pharmacologique (208) est configuré pour être contenu dans la seringue (238), dans lequel l'introducteur (204) est configuré pour recevoir la seringue (238) afin de faciliter l'administration de l'agent pharmacologique (208) au nerf cible.
